# EUROPEAN PATENT SPECIFICATION

(11) **EP 2 952 886 B1**
(45) Date of publication and mention of the grant of the patent: **23.09.2020**
(21) Application number: 14171633.2
(22) Date of filing: 06.06.2014
(51) Int. Cl.: G01D 11/24, G01N 33/00, H01L 23/31, G01N 27/12

(54) **Method for manufacturing a gas sensor package**
Herstellungsverfahren für Gassensorpaket
Procédé de production d'un boîtier pour capteur de gaz

(43) Date of publication of application: 09.12.2015
(73) Proprietor: Sensirion AG, 8712 Stäfa (CH)
(72) Inventor: Mayer, Felix, 8712 Stäfa (CH); Hunziker, Werner, 8712 Stäfa (CH); Pustan, David, 8712 Stäfa (CH); Bühler, Johannes, 8712 Stäfa (CH)

(56) References cited:
- WO-A1-2009/084871
- DE-U1-202011 051 190
- US-A1- 2013 056 703
- US-A1- 2014 084 390

## Description

### Field of the Invention

The present invention relates to a method for manufacturing a gas sensor package.

### Technical Background

An increasing number of gas sensors tend to be integrated into semiconductor chips. In view of specifics of the gas sensors as such, a method for manufacturing a gas sensor package is desired that addresses these specifics.

DE 202011051190 U1 discloses a sensor module, comprising an electrically conductive structure, a sensor chip disposed on the electrically conductive structure and electrically connected thereto, an encapsulation for the sensor chip in such a way that the sensor module has a cuboid shape, an access in the encapsulation to a surface portion of the sensor chip, which access is arranged centrally on a top side of the sensor module, a bottom side opposite of the top side of the sensor module, the bottom side having areas bare of the encapsulation which expose the electrically conductive structure, which areas comprise a central area and four edge areas.

WO 2009084871 A1 discloses a micro gas sensor and a method for fabricating the same that comprises a micro heater formed inside a polysilicon membrane by doping impurities into a specific region of the polysilicon membrane positioned under a gas sensing substance, thereby improving thermal structural stability and making it easy to form the gas sensing substance. The micro gas sensor comprises: a micro heater formed by doping impurities into polysilicon vapor-deposited on a substrate on which a first insulating layer is formed; a polysilicon membrane for decreasing a heat loss of the micro heater; a power electrode for supplying power and a temperature measurement electrode for measuring a temperature, positioned at both ends of the micro heater; a second insulating layer formed on the micro heater; a sensing substance formed on the second insulating layer, for sensing a gas; and a sensing electrode for measuring a change in properties of the sensing substance. The method for fabricating a micro gas sensor comprises steps of: forming polysilicon on a substrate on which a first insulating layer is formed; forming a micro heater by doping impurities into the polysilicon; forming electrodes at both ends of the micro heater; forming a second insulating layer on the micro heater; forming a sensing substance on the second insulating layer; and forming a sensing electrode on the sensing substance.

### Disclosure of the Invention

A method for manufacturing a gas sensor package is provided comprising the steps of mounting a semiconductor chip on a carrier and applying a molding compound for at least partially enclosing the semiconductor chip thereby generating an opening in the molding compound. The opening provides access to a portion of the semiconductor chip being uncovered by the molding compound. A sensitive material is applied through the opening onto the uncovered portion of the semiconductor chip for building a layer sensitive to a gas.

Preferably, multiple gas sensor packages are manufactured in the same processing steps thereby making use of a carrier onto which multiple semiconductor chips can be placed. After the semiconductor chips being mounted to the common carrier, the molding compound is applied to the carrier and thereby to each semiconductor chip on the carrier. After molding, the sensitive material is applied through each opening onto the uncovered portion of respective semiconductor chip for building a layer sensitive to a gas. The sensitive material may be applied by contactless dispensing, such as ink jet printing, for example. It is not required that the entire portion uncovered by the molding compound is filled by the sensitive material. The sensitive material may be applied to a section of this portion. Summarizing, the sensitive layer is built after the package is processed by molding.

This order of processing steps - i.e. manufacturing the sensitive layer through the opening of the package after having processed the package - is preferred over a reversed order in that an already built sensitive layer would require protection during the manufacturing of the package, i.e. during molding. In case the sensitive layer were manufactured prior to the package and no protection means would be installed, the molding process and / or other processing steps in between would affect the sensitive layer, e.g. causing mechanical damage thereto, or impacting the sensing characteristics in case particles stemming from the molding process attach to or migrate into the sensitive layer. This is in particular the case, when the sensitive layer is a porous layer, such as a layer comprising metal oxide material. Unlike with other sensors that need no direct access to the environment, a gas sensor preferably requires such access in form of an opening in the molding compound. Hence, in case it is molded prior to applying the sensitive material, the opening in the molding compound that is formed anyway according to the present embodiment is now a dual-use opening through which not only the gas reaches the sensitive element during measuring operations, but also through which the sensitive material is applied to the semiconductor chip for building the sensitive layer.

This order of manufacturing steps also provides benefits in handling the device. In case the dispensing of the sensitive material may be executed at a by a different tool and / or at a different place than the mounting of the semiconductor chips on the carrier, the device preferably is transferred from one location to another one by a picker. In case the molding compound is already applied, the picker may pick up the device at the molding compound rather than at an unprotected semiconductor chip. This may be true in any further handling of the device with the individual semiconductor chips still being on the common carrier, or even later after separating the individual gas sensor packages from each other.

Hence, the early adoption of the molding compound facilitates handling and increases protection during further manufacturing steps and beyond. In a particular embodiment, the openings may be closed temporarily e.g. by a membrane or a tape for protecting the sensitive layers inside the openings.

In the above order of manufacturing steps, major portions of the semiconductor chip are also protected by the molding compound during dispensing the sensitive material.

The sensitive layer is made from material being sensitive to one or more analytes. The sensitive layer may comprise multiple individual layer sections arranged next to each other and being separated from each for building a sensor array comprising a set of sensor cells, wherein a sensor cell may be understood as an entity of the gas sensor which may be read individually. Preferably, in the embodiment of the sensor array, each or at least some of the layer sections are suitable for sensing analytes, and in particular different analytes. Analytes may include one or more of, for example, H2O, CO2, NOX, ethanol, CO, ozone, ammonia, formaldehyde, or xylene without limitation. Specifically, the sensitive layer may contain a metal oxide material, and in particular a semiconducting metal oxide material, and specifically may contain metal oxide materials of different composition per layer section. A metal oxide material generally may include one or more of tin oxide, zinc oxide, titanium oxide, tungsten oxide, indium oxide and gallium oxide. Such metal oxides may be used for the detection of analytes such as VOCs, carbon monoxide, nitrogen dioxide, methane, ammonia or hydrogen sulphide. Metal oxide sensors are based on the concept that gaseous analytes interact with the metal oxide layer at elevated temperatures of the sensitive layer in the range of more than 100° Celsius, and specifically between 250°C and 350°Celsius. As a result of the catalytic reaction, the conductivity of the sensitive layer may change which change can be measured. Hence, such chemical sensors are also denoted as high temperature chemoresistors for the reason that a chemical property of the analyte is converted into an electrical resistance at high temperatures of the sensitive layer. Preferably, by means of such a gas sensor a gas may be investigated at least as to the absence or presence of the subject analyte/s the gas sensor is sensitive to. Hence, the gas supplied to the gas sensor may be analyzed by means of the sensitive layer as to if and which of the chemical substances or compounds the sensitive layer is sensitive to are present in the gas supplied. A combination of analytes detected in the gas supplied may suggest for a certain odour or for a certain gas. It is always subject to a design of the gas sensor as to how many different analytes and/or how many different properties of an analyte the gas sensor is sensitive to.

In another embodiment, the sensitive material may comprise a polymer that in one embodiment may be sensitive to H₂O such that the sensor may be a humidity sensor. A capacity or a resistance of such polymer layer may be measured for deriving information as to the gas that may interact with the sensitive layer.

The sensitive material may, after being applied to the semiconductor chips, need to be annealed by applying heat thereto. Rather than separating each gas sensor package from the present assembly and individually applying heat to each sensitive layer, it is envisaged in a very preferred embodiment to anneal the sensitive layer of the gas sensor chips when still residing on the common carrier, i.e. prior to separating the gas sensor packages from each other. However, conventional annealing steps such as exposing the carrier with the gas sensor packages to heat generated externally, such as in an oven, may not be applicable in view of the molding compound possibly melting when being exposed to such heat. Hence, it is preferred that heat is locally applied to the sensitive layers without affecting the molding compound or other organic material. This can be achieved by supplying an electrical current to heaters that in one embodiment are provided anyway in each semiconductor chip when such heater is required for operating the gas sensor chip, i.e. when the sensitive layer needs to be heated to a certain temperature for enabling a measurement. In this case, the heater of each semiconductor chip may be supplied with electrical current for annealing the associate sensitive layer / the sensitive material.

In one embodiment, the carrier for the various semiconductor chips at least comprises an electrically conducting leadframe which is understood as an electrically conducting structure of leads made out of metal, for example, by stamping or etching from a metal plate. When multiple gas sensor packages are to be fabricated on a single carrier, such leadframe preferably comprises a die pad per semiconductor chip for mounting the semiconductor chip to, which die pad is understood as a flat portion of the leadframe. Multiple die pads may be provided per gas sensor package in case other chips are to be arranged in the same gas sensor package, such as an ASIC, etc. In another embodiment, the other chips are arranged on the same die pad as the semiconductor chip representing a gas sensor chip. In addition, the leadframe comprises contact pads for each gas sensor package to be manufactured for electrically contacting the gas sensor packages later on. In order to provide a single piece carrier, support leads are provided in the leadframe for connecting to the die pads and the contact pads. In case of each semiconductor chip comprising a heater, it is preferred that one of the contact pads of each gas sensor package is configured as a heater pin for supplying an electrical current to the heater while another contact pad serves as a pin for supplying power to the gas sensor package except for the heater.

When now annealing the sensitive layers by means of the associate heaters it is desired to selectively apply the heating current solely to the contact pads representing the heater pins. However, at the present stage of manufacturing, the leadframe still provides an electrically conducting structure reaching all contact pads and die pads. Applying a current in magnitude and time as is required for annealing purposes to every contact pad is not desired since it may affect and / or damage the semiconductor chips that are not dimensioned to be operated with such a high heating current.

Hence, it is desired to electrically isolate all the contact pads from each other. For doing so, it is preferred to sever support leads where necessary in order to electrically decouple the contact pads from each other and from the rest of the leadframe.

The electrically decoupling of the contact pads preferably is achieved by severing support leads connecting the contact pads, which comprises mechanically interrupting these support leads. The severing step preferably is applied after applying the molding compound and after applying the sensitive material. The severing step preferably is applied to the support leads connecting the contact pads or the contact pads themselves from the back side of the present device, where typically portions of the leadframe are accessible. The front side of the present device preferably is attached to a dicing tape during dicing thereby covering and protecting the openings in the molding compound.

Preferably, each die pad has a rectangular shape each with four edges, wherein the contact pads are arranged at opposite edges of the corresponding die pad. The support leads connecting each die pad instead depart from the die pad at least from one of the other two edges. This enables severing the contact pads or respective support leads arranged at one edge of the corresponding die pad by a single severing step, such as a single sawing step. At this stage, it is preferred that the support leads connecting the die pads are not severed yet but only in a final dicing step for separating the gas sensor packages after having annealed the sensitive layers.

In a preferred embodiment, the locations to be severed may e.g. be marked, e.g. by etching.

After having prepared the carrier, and in particular the leadframe of the carrier in such way, a heating current may be applied to one or more of the contact pads serving as heater pins thereby heating the heaters of the semiconductor chips, e.g. for annealing purposes.

After annealing, the gas sensor chips may be tested and / or calibrated while still being mechanically coupled via the common carrier, e.g. by applying probes to the contact pads. Finally, the carrier, and specifically the leadframe, and possibly the molding compound may be diced for separating the gas sensor packages. In one embodiment, a dicing tape is attached to the front side of the present device thereby covering the openings in the molding compound. The device then is diced into individual gas sensor chips from the back side. In this way, the sensitive layers accessible through the openings are protected during dicing such that no water or diced material may impact the sensitive layers.

As to the semiconductor chips, it is preferred that prior to mounting the or each semiconductor chip onto the carrier, the or each semiconductor chip is prepared by integrating a processing circuit to a front side of the semiconductor chip - which processing circuit preferably is configured for pre-processing signals from the sensitive layer and for controlling the heater -, integrating the heater to the front side of the semiconductor chip, and generating a recess in a back side of the semiconductor chip underneath the heater thereby generating a membrane in the semiconductor chip. The recess may be manufactured by etching such as dry- or wet-etching, or otherwise removing material from the backside of the semiconductor chip, such as bulk substrate material. The remaining material of the semiconductor chip above the recess forms a membrane which may include e.g. CMOS layers and / or parts of the bulk substrate material. Such recess serves for thermally isolating the membrane from the rest of the semiconductor chip which preferably carries the processing circuit. Given that the sensitive layer will be applied to the membrane and the heater resides on or in the membrane, heat generated by the heater can only to a limited degree migrate into the other portions of the semiconductor chip. The molding compound is preferably not applied to the membrane in order not to be in touch with this heated portion. After having mounted the gas sensor chip to the die pad, the recessed portion of the semiconductor chip and the die pad form a cavity underneath the membrane. Heat generated by the heater in the membrane during operation may be transferred via gas in the cavity to the die pad acting as a heat sink. The die pad preferably is made from an electrically and thermally conducting material, such as metal, as is the entire leadframe. The semiconductor chip may be attached to the die pad in one example such that a substrate of the gas sensor chip may be grounded via the die pad. The die pad may have a footprint approximately equal to a footprint of the semiconductor chip. In one embodiment, a hole or another sort of channel is manufactured in the die pad for venting the cavity there through in the context of pressure balancing, and for removing moisture from the cavity. The hole may be fabricated subject to the material of the die pad and/or the carrier in general, e.g. by etching, piercing, laser drilling, mechanical drilling, etc.

In a preferred embodiment, after mounting the or each semiconductor chip to the carrier comprising the contact pads and prior to applying the molding compound, the or each semiconductor chip is electrically connected to the assigned contact pads, either directly or via a separate ASIC, preferably by means of wire bonding.

In an alternate embodiment, the carrier may be a printed circuit board with the contact pads being formed by metallizations on a front side of the printed circuit board, and the contact pads are formed by metallizations on a backside of the printed circuit board which additionally requires vias through the printed circuit board for connecting the contact pads. Instead of a printed circuit board, another carrier such as a ceramic substrate or a glass substrate may be used.

The molding compound which preferably is an epoxy with filler particles which filler particles e.g. may be glass, and specifically SiO2, and which is applied to the or each semiconductor chip prior to applying the sensitive material is preferably configured to at least partially enclose and/or encapsulates the semiconductor chip. In case of the manufacturing for multiple gas sensor packages, the molding compound is applied to all semiconductor chips on the common carrier in a common manufacturing step thereby generating a continuous molding compound across the common carrier, including encapsulation of other chips if any.

In another embodiment, individual molding compound blocks are molded for the future gas sensor packages, the molding compound blocks only being connected to each other via support leads of the leadframe.

The opening in the molding compound allows a gas to be measured to access the sensitive layer on the semiconductor chip collectively forming a gas sensor chip. At the same time, the opening allows to build the sensitive layer there through.

After having applied the sensitive material, the molding compound encapsulates and as such covers the gas sensor chip essentially except for the sensitive layer such that any outgassing from the gas sensor chip itself, from adhesives between the gas sensor chip and the carrier, or from the carrier itself does not have any impact on the measurement because these elements do not share a common volume with the sensitive layer. Such common volume is also referred to as dead volume within such package, and an outgassing from any of these elements, in particular in view of the heating required for operating the sensitive layer, may affect the measurement temporarily, or even permanent. However, in the present embodiment, the dead volume is reduced and limits any outgassing effect on the sensitive layer. Hence, it is preferred, that the sensitive layer on the one hand and on the other hand the carrier, the gas sensor chip and any adhesive in between do not share a common volume.

For manufacturing the package with the opening, a mold used in the molding process may have a protrusion. In this case, the gas sensor chip may be protected from mechanical impact during molding and the area for the designated sensitive layer may be sealed by a preferably elastic layer arranged on top of semiconductor chip in the area and / or arranged at the mold at the protrusion or at least parts thereof. After the molding, the layer may be removed again. In a different embodiment, a sealing frame may be deposited around an area the sensitive layer is expected to be arranged at. The protrusion of the mold for manufacturing the opening may then sit on the stress relief frame during molding. The sealing frame may not necessarily be removed after molding. The sealing frame may be made from elastic material.In another embodiment, a wall element may be arranged on top of the gas sensor chip encircling the area the sensitive layer is expected to be arranged at. Such wall element may, for example, either be bonded to the gas sensor chip or be manufactured by photolithographic steps, may be of a material different to the molding compound, and may act as a barrier preventing the molding compound from entering the area designated for the sensitive layer. In this embodiment, the mold may not necessarily need a protrusion but even can be flat and directly sit on the wall element during molding, e.g on a sealing layer thereof. The wall element may not be removed after molding but continues to define the opening in the molding compound given that the molding compound stops at the wall. The wall preferably is of a material different than the molding compound.

In this embodiment, the gas sensor chip may be bonded to the die pad, and such device may be molded with the molding compound not only serving as partial encapsulation of the gas sensor chip but also serving as mechanical fixture for the contact pads and the die pad.

According to another aspect of the present invention, a gas sensor package is provided the gas sensor package being manufactured according any of the previous embodiments of the method. In the gas sensor package, it is envisaged that the heater is not supplied with power via the contact pad serving as power supply for the sensor chip. Instead, the heater is supplied with power via a dedicated contact pad serving as heater pin. This contact pad may, via the electrical connection and metallizations in the gas sensor chip directly be connected to the heater. Given that the heater may demand currents of e.g. more than 10 mA and possible up to 100 mA, it was found beneficial that the power supply for the regular operation of the gas sensor chip, which may be supplied by a current in the order of few mA, such as 1.8 mA, may be separated from the power supply for the heater. In this way, overall reliability of the operation of the gas sensor package is enhanced. For reliably measuring gas, a stable and precise power supply is required for the gas sensor chip and preferably for its processing circuit. If the current for the heater were to be derived in the gas sensor chip from the normal power supply, the normal power supply may show fluctuations which are not desired. In a preferred embodiment, the contact pad serving as pin for operating the heater and the other contact pad serving as supply pin for supplying power for operating the gas sensor chip except for the heater are spaced apart from each other, and e.g. are arranged at different edges of the back side of the gas sensor package. In this way, any heating of the gas sensor chip and its environment is introduced from different ends and does not accumulate in a hot spot if the contact pads were neighbors. In an embodiment, the processing circuit integrated in the gas sensor chip or provided in form of a separate ASIC may control the heating of the heater.

It is preferred that the opening in the molding compound is arranged in a front side of the gas sensor package while the contact pads and the die pad are arranged at a back side thereof. This provides a compact small size package suited for being arranged into portable electronic devices such as mobile phones, tablet computers, etc. In this context, the gas sensor package may in a preferred embodiment have the shape of a cuboid. The opening in the molding compound preferably has a circular footprint and may in one embodiment be centered in the front side of the gas sensor package, and in another embodiment be arranged off-centered in the front side. The geometry of the access opening mainly is dependent on the size of the dimensions of the membrane the sensitive layer is to be deposited to in one embodiment, such that in an effort to minimize the overall size of the gas sensor package, it is desired to reduce the footprint of the molding compound outside the opening if possible. However, it is also desired to keep the footprint of the access opening as small as possible since the volume defined by the opening may be considered as dead volume for the sensitive layer and any outgassing of the molding compound affecting the sensitive layer may be reduced the smaller the molding compound surface defining the dead volume is. Generally, such small package size is not only desired in view of the application of such gas sensor package in portable electronic devices, but also in view of a possible manufacturing in which the sensitive layer may be applied to the gas sensor chip through the opening of the already manufactured molding compound. In case the gas sensor packages are still residing on a common carrier during this manufacturing step, any working tool applying the sensitive layers to the gas sensor packages operates quicker the smaller a travel distance is between the openings of adjacent gas sensor packages. The molding compound in turn provides a mechanical stability for the gas sensor packages when applying the sensitive layers, e.g. by ink jet printing since the gas sensor chip including the membrane are already fixed within the molding compound. In addition, a small package size is beneficial in that the dead volume is reduced and limits any outgassing effect on the sensitive layer.

Hence, in a preferred embodiment, the cuboid gas sensor package has a footprint with a length l times a width w in mm²,. In particular, the cuboid gas sensor package has a footprint l x w mm² with l ∈ [2.3, 2.6] mm, w ∈ [2.3, 2.6] mm. It is preferred that the diameter d of the opening is less than 2 mm, and in particular d ∈ [1.4, 1.6] mm. In case the opening varies in diameter down to the gas sensor chip, and e.g. narrows, it is assumed that the diameter d is the maximum diameter of such opening, which in the latter case would be the diameter in the top surface of the molding compound. All these embodiments aim at a small size gas sensor package while at the same time limiting the dead volume relevant for the impact of outgassing and allowing a sufficient thermal insulation of the sensitive layer.

In one embodiment, the gas sensor package has six contact pads. As indicated above, a first one of the contact pads serves as pin for supplying a current to the heater. Another one of the contact pads may serve as a supply pin for supplying power for operating the gas sensor chip except for the heater. A third one of the contact pads serves as a ground pin. A fourth one of the contact pads serves as a data pin for at least receiving measurement data from the gas sensor chip communicated according to a communication protocol. A fifth one of the contact pads serves as pin for a clock for operating the communication protocol, such as the I2C protocol. A sixth one of the contact pads serves as a programmable pin for programming the gas sensor chip.

Preferably, the six contact pads are arranged in two rows by three at the backside of the gas sensor package and the die pad is arranged between the two rows of three contact pads each. This allows for a dense arrangement, in particular if the die pad is of rectangular shape.

In a preferred embodiment, the front side of the gas sensor package comprises at least one marking. In case the footprint of the gas sensor package is of rectangular shape each marking is arranged in a corner of the front side. Preferably, a single marking is applied to one of the corners for defining an orientation of the gas sensor package.

In the present application, molding compound or pre-molding compound shall at least include any plastic material or dry resist that may in any form be molded, such as injection-molded, transfer-molded, or otherwise molded.

The present gas sensor package in its various embodiments not only is small in its dimensions but at the same time reduces outgassing possibly impacting measurements, ensures a stable / "clean" power supply V_{DD} while at the same time supplying a high current to the heater not interfering with the power supply for the gas sensor chip. The heating of the sensitive layer is only locally applied, where temperatures of more than 200 ° degrees Celsius and sometimes even far more may be achieved. In contrast, outside the membrane with the heater and the sensitive layer the temperature may not exceed e.g. 85 ° degrees Celsius for avoiding an impact on processing sensor signals. A corresponding processing circuit preferably is arranged outside the membrane and preferably supplies digital signals to one of the contact pads. The present gas sensor package preferably is finally can be SMD mounted to an external support. In this process step, it is beneficial that the gas sensor package can be taken by a picker at the mold without causing damage to the semiconductor chip.

Other advantageous embodiments of the gas sensor package are listed in the dependent claims as well as in the description below.

### Brief Description of the Drawings

Embodiments of the present invention, aspects and advantages will become apparent from the following detailed description thereof. Such description makes reference to the annexed drawings, wherein the figures show:
Fig. 1 in diagrams a) to c) manufacturing steps as applied in an embodiment of a method according to the present invention;
Fig. 2 in diagrams a) to e) manufacturing steps as applied in an embodiment of a method according to the present invention; and
Fig. 3 a gas sensor package according to an embodiment of the present invention, in a perspective view in diagram a), in a top view in diagram b), in a bottom view in diagram c), and in a cut in diagram d).

### Detailed Description of the Drawings

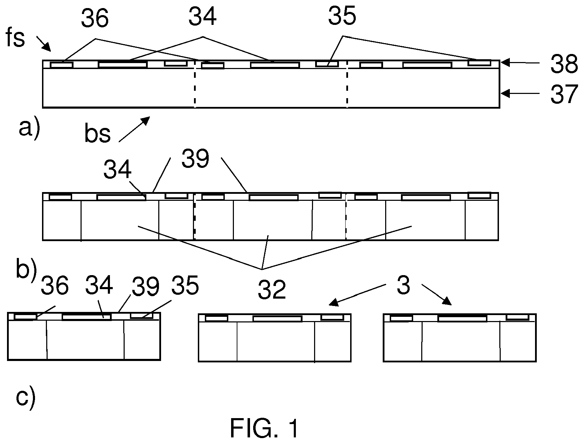

Figure 1 illustrates in diagrams a) to c) manufacturing steps as applied in a method for manufacturing a gas sensor package according to an embodiment of the present invention. These steps refer to the manufacturing of suitable semiconductor chips that later will packaged into gas sensor packages. In diagram 1 a), a wafer is provided containing a semiconductor substrate 37 and a layer stack 38 arranged on the semiconductor substrate 37 at a front side fs of the wafer. The wafer as such may be a standard CMOS wafer into which integrated processing circuits 36 are manufactured, as well as contact pads 35, for example. The vertical dashed lines in the wafer indicate dicing trenches along which the wafer will be diced into multiple semiconductor chips later on. In addition to the bond pads 35 and processing circuits 36, heaters 34 are manufactured in the front side fs of the wafer, e.g. embodied in one of the patterned metal layers of the layer stack 38.

After having prepared the wafer according to diagram 1 a), recesses 32 are manufactured into a backside bs of the wafer, and specifically underneath each heater 34. The recesses 32 may in one embodiment be manufactured by etching into the semiconductor substrate 37. By doing so, each future semiconductor chip comprises a membrane 39 that may be built from the layer stack 38 and possibly from a thin portion of the semiconductor substrate 37. The membrane 39 preferably serves for thermal insulation.

According to diagram 1 c), the wafer then may be is diced into individual semiconductor chips 3, each semiconductor chip 3 comprising a membrane 39 at its front side fs, and a heater 34 deposited on or integrated into the membrane 39, a processing circuit 36 and bond pads 35 for electrically contacting the semiconductor chip 3, both preferably arranged outside the membrane 39.

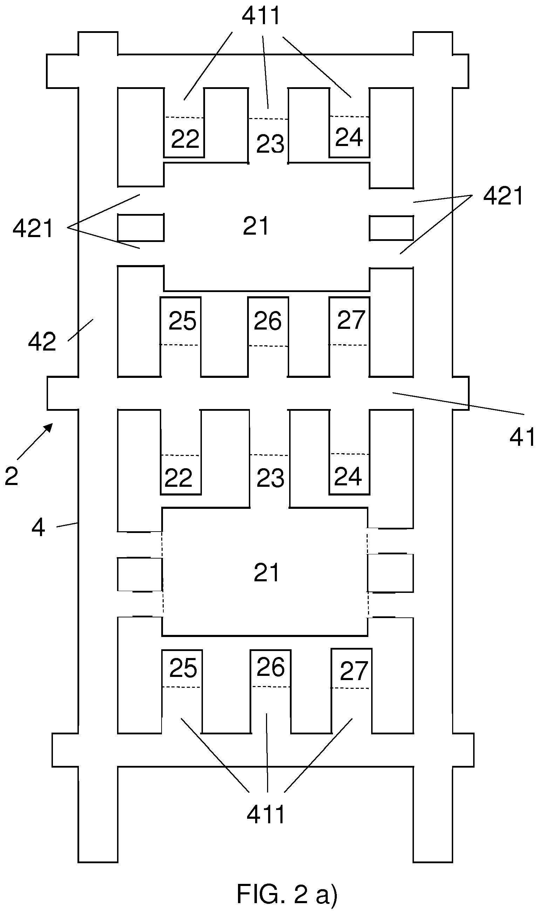

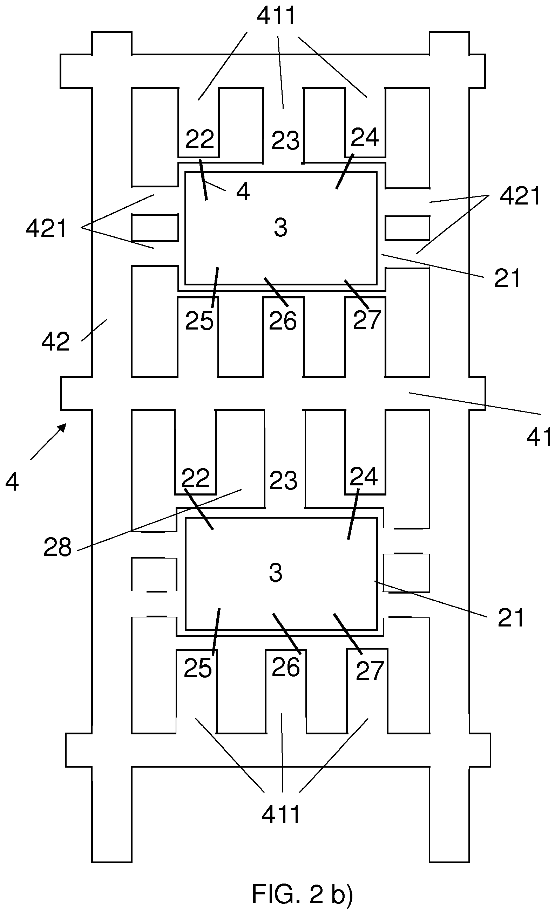

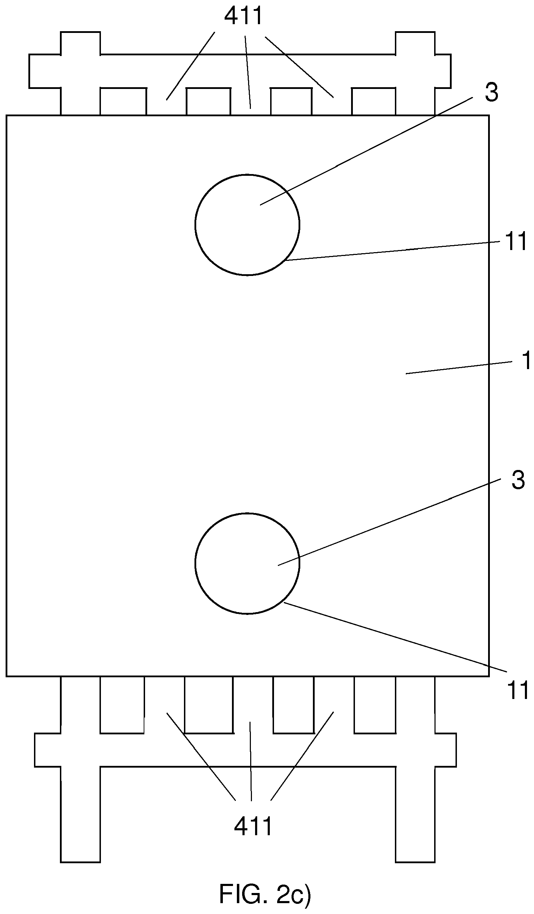

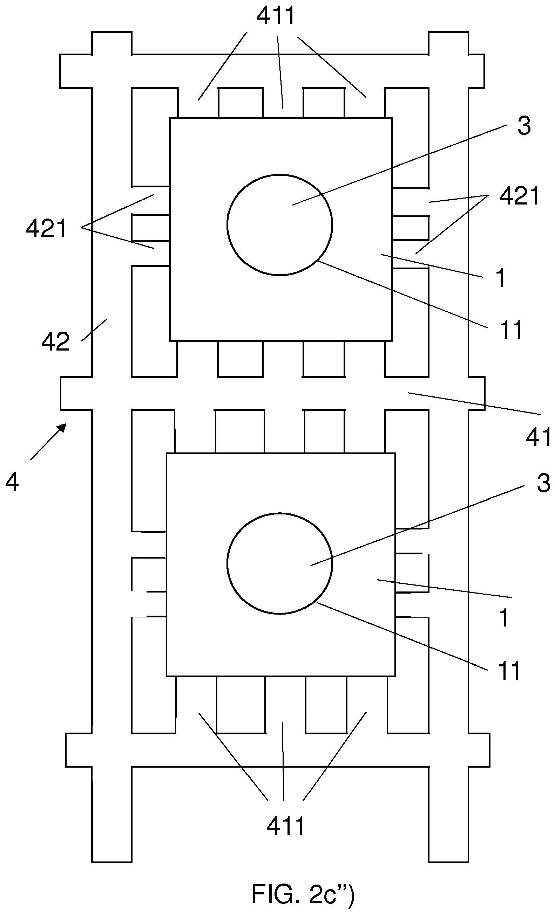

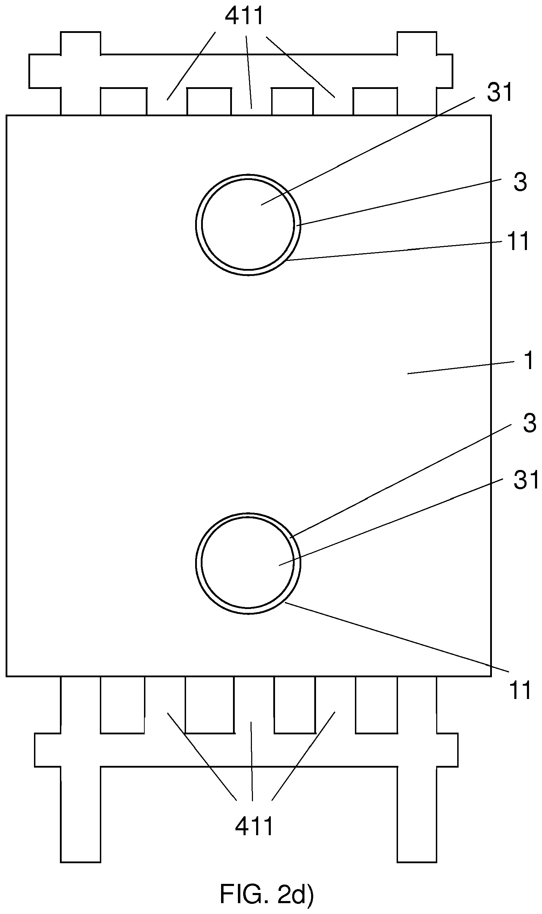

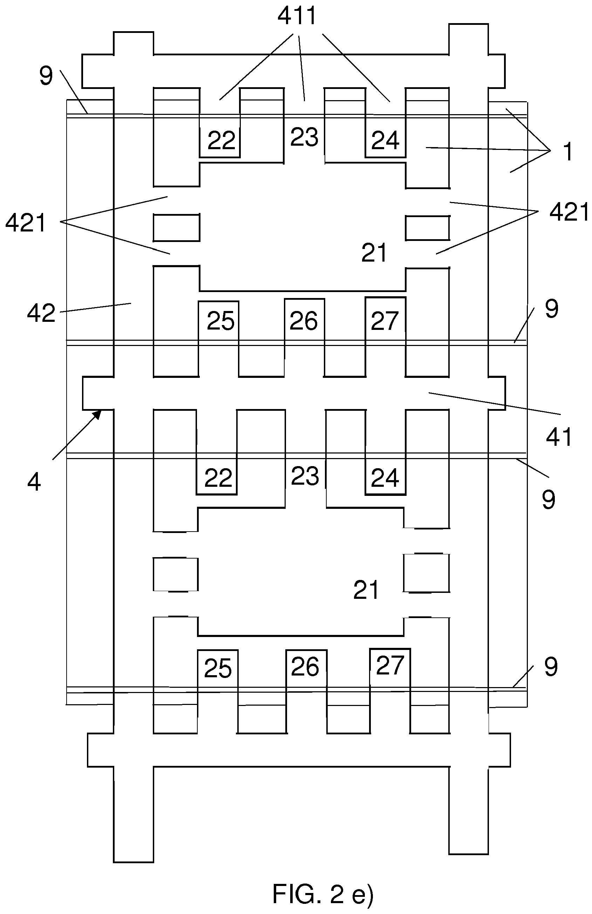

Figure 2 illustrates in diagrams a) to h) manufacturing steps as applied in a method according to an embodiment of the present invention.

According to diagram 2 a), a leadframe 4 is provided a cutout of which is shown in diagram 2 a). A leadframe typically is a grid like structure made from an electrically conducting material. The individual interconnected leads may be etched or stamped from a thin plate of metal. It is assumed, that the leadframe 4 already is prepared to a shape as shown in diagram 2 a) for the purpose of manufacturing a set of gas sensor packages thereon. Although in the present example, the leadframe 4 comprises only two platforms for building a gas sensor packages on, it is understood, that in real manufacturing the leadframe 4 may contain many more platforms, e.g. for building tens or hundreds of sensor packages on. Hence, the leadframe 4 contributes to a carrier 2 that is common to all the gas sensor packages which are desired to be manufactured in a common manufacturing process.

The present leadframe 4 contains, for example, horizontal support leads 41 and vertical support leads 42. The leadframe 4 further comprises die pads 21, each die pad 21 serving as a platform to mount a semiconductor chip on. Each die pad 21 is connected to the vertical support leads 42 by means of support leads 421 which may also be referred to as dies pad supports. The horizontal support leads 41 contain finger like extensions, the end sections of which extensions represent contact pads 22-27 of the future gas sensor packages. Those end sections are indicated by dashed lines, while the extensions as such are referred to as support leads 411.

In a next step, as is shown in diagram 2 b), a semiconductor chip 3 is mounted on each die pad 21. The semiconductor chips 3 may be the semiconductor chips prepared according to Figure 1. Each semiconductor chip 3 may be placed on the corresponding die pad 21 with its back side bs facing the die pad 21, such that a cavity is built from the recessed portion of the semiconductor chip 3 and the die pad 21. In a next step, the semiconductor chip 3 is electrically connected to the contact pads 22-27 by wire bonding as is indicated by bond wires 4.

In a step according to diagram 2 c), a molding compound 1 is applied on top of the present structures such that the molding compound 1 encapsulates the corresponding semiconductor chips 3 except for an opening 11 which opening 11 provides access to a portion of the semiconductor chip 3 which preferably is the membrane 39. The molding compound 1 provides mechanical stability and protects the semiconductor chip 3. The molding compound 1 preferably is made from insulating material, such as epoxy containing filler particles such as glass, specifically SiO2. For manufacturing the molding compounds 1 the device of diagram 2 c) is inserted into a mold. The mold is then filled, e.g. by a liquid molding compound. Thereafter, the molding compound 1 may harden into a solid molding compound 1 such as referred to in diagram 2 c). As can be seen from this diagram, the molding compound extends over the carrier 2 into a continuous molding compound except for the openings 31. In a different embodiment as shown in diagram 2 c"), the molding compound 1 is applied into individual blocks per future gas sensor package which blocks are disconnected from each other. For manufacturing these molding compound blocks, the mold has a different shape than for producing the mold compound of diagram 2c).

In the step shown in diagram 2 d), a sensitive material is applied through each opening 11 and onto the respective portions of the semiconductor chips 3 for forming a sensitive layer 31. The sensitive material may be contactless dispensed into the opening 11, e.g. by means of the print head of an ink jet printer that supplies sensitive material in form of a solution or suspension.

The sensitive material may, after being applied to the semiconductor chips 3, be annealed by means of heat. Rather than separating each gas sensor package from the present assembly and individually applying heat to each sensitive layer, it is envisaged to anneal the sensitive layer 31 of all gas sensor chips while still residing on the common carrier 2, i.e. prior to dicing the molding compound 1 and the carrier 2 into individual gas sensor packages. In one embodiment, it is preferred to locally heat the sensitive layers 31 by means of the heaters 34 integrated in the semiconductor chips 3 since there may be scenarios in which the mold compounds 1 would not stand and an external heating of the entire device.

When annealing by means of the heaters 34, the present device does not yet offer a selective application of a heating current to the heaters only, since applying a voltage or a current to the present leadframe 4 would rather end up at every contact pad. In view of high magnitudes of the heating current and a long duration of an annealing process, it is desired to apply the heating current only to those contact pads connected to the heaters. For this reason, it is desired to electrically isolate all contact pads 22-27 from each other. For doing so, it is preferred to sever either the supply leads 411 connecting to the contact pads 22-27, or the contact pads 22-27 themselves which rather is a question of definition.

For severing supply leads for the above purpose, it is preferred to sever these supply leads from the back side of the device which present back side is shown in diagram 2 e). The result can be seen in the bottom view according to diagram 2 e). Sawing lines 9 are indicated along which the support leads 411 of the contact pads 22-28 of each gas sensor package will be severed. Instead of two slim sawing lines 9 as shown in diagram 2 e), a wide sawing line can be provided between two gas sensor packages for severing the contact pads assigned to two different die pads in a single sawing step. The support leads 421 connecting the die pads 21 remain intact, i.e. it is only horizontally sawed. After the sawing step, current may be applied to the contact pads 26 and the support leads 42 which connect via the die pads 21 to the contact pads 23 serving as ground pins. By doing so, the sensitive layers 31 including the sensitive material may be annealed.

In further steps, the gas sensor packages on the common carrier 2 may be calibrated and / or tested, e.g. by applying electrodes to the contact pads 22-27 from the back side.

In a final step, the gas sensor packages may be separated from each other by dicing the leadframe 4 and the molding compound 1, preferably from the back side and after the device is attached to a dicing tape with its front side for protecting the openings

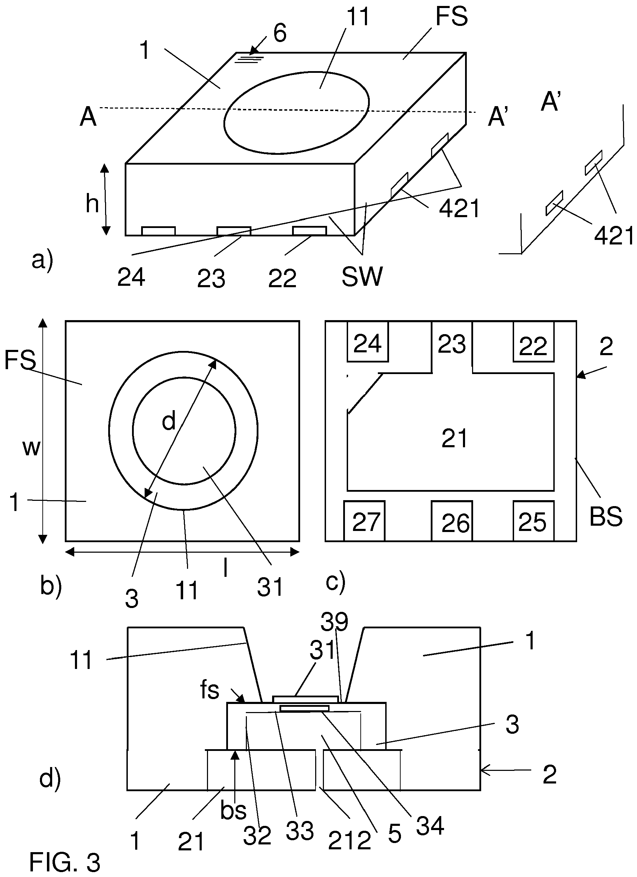

Figure 3 illustrates an individual gas sensor package according to an embodiment of the present invention which preferably is manufactured according an embodiment of a manufacturing method, and specifically according to the manufacturing method illustrated in Figure 2. Diagram 3 a) illustrates the gas sensor package in a perspective view, diagram 3 b) in a top view, diagram 3 c) in a bottom view, and diagram 3 d) in a cut view along lines A-A' of diagram 3 a).

The gas sensor package has the shape of a cuboid which is defined by a molding compound 1 and which has a front side FS and a back side BS opposite to the front side FS. An opening 11 in the molding compound 1 provides access to a sensitive layer 31 of a gas sensor chip 3. In one of the corners of the molding compound 1 on the front side FS, a marking 6 is provided, e.g. by laser processing, ink jet printing, etc. The marking preferably serves for indicating an orientation of the gas sensor package, but may instead or additionally also indicate one or more of a device number, a serial number, the manufacturer, etc. In side walls SW, front ends of contact pads 22, 23, 24 are exposed from the molding compound 1 as well as front ends of support leads 421.

However, in a different embodiment, one or more of the contact pads and/or the die pad supports 211 may not finalize with the bottom edge of the gas sensor package as shown in Figure 1, but may be elevated from such edge such as shown in the cutout to the right of Figure 1, where the die pad supports 211 are arranged at a distance of e.g. between 100 µm and 200 µm from the bottom edge.

The gas sensor package of the present example has a height h of preferably between 0.7 and 0.8 mm. The opening 11 is of circular shape and has a diameter d, less than 2 mm, and preferably between 1.4 and 1.6 mm. The gas sensor package has a footprint l x w with each of the length l and the width preferably being in the range between 2.3 and 2.7 mm. The sensitive layer 31 preferably has a diameter little smaller than the diameter d of the opening such that a small portion of the gas sensor chip 3 outside the sensitive layer 31 is visible in the top view.

The back side BS of the gas sensor package, see diagram 4 c), shows six contact pads 22-27. The contact pads 22-27 are arranged in two rows of three contact pads each at two opposite edges of the gas sensor package. Preferably, each contact pad is dimensioned by less than 0.5 mm by 0.5 mm, and a pitch between each two contact pins of a row is in the range of 0.8 mm. Preferably, the contact pad 26 serves as heater pin for supplying electrical current to the heater. Contact pad 24 serves as a supply pin for supplying power for operating the gas sensor chip 3 except for the heater. Contact pad 23 serves as a ground pin. Contact pad 22 serves as a data pin for at least receiving measurement data from the gas sensor chip communicated according to a communication protocol, such as the I2C protocol. Contact pad 27 serves as pin for a clock for operating the communication protocol, such as the I2C protocol. Contact pad 25 serves as a programmable pin for programming the gas sensor chip 3, e.g. with calibration data. In between the two rows, a die pad 21 is provided which serves as support for the gas sensor chip 3. The die pad 21 is of rectangular shape and has one flattened corner which may serve as an optical and/or mechanical encoding for an orientation of the gas sensor package. The contact pads 22-27 and the die pad 21 are mechanically linked by the molding compound 1.

According to the cut view according to diagram 4 d), the gas sensor chip 3 is arranged on top of the die pad 21, and e.g. is bonded thereto. The gas sensor chip 3 has a front side fs and a back side bs. In one embodiment, the gas sensor chip 3 may comprise a semiconductor substrate and CMOS layer arranged on top of the substrate. The substrate may be etched or otherwise partially removed from the backside bs such that the gas sensor chip 3 has a recess 32 on its backside bs. As a result of building the recess 32 in the gas sensor chip 3, a thinned structure is generated in the gas sensor chip 3, also referred to as membrane 39. The sensitive structure 31 is arranged on or in the membrane 39.

In a specific embodiment, the sensitive layer 31 comprises a metal oxide layer which is to be heated for enabling the sensing of chemical analytes. For this purpose, a heater 34 such as a resistive heater is arranged in or under the membrane 33 for heating the sensitive layer 31. Hence, both the gas sensitive layer 31 and the heater 34 may be arranged on or in the membrane 39 above the recess 32. This arrangement is owed to a thermal insulation the membrane 39 provides which improves the accuracy of the measurement.

In a preferred embodiment, the die pad 21 has a hole 212 for connecting the cavity 5 to the outside world. Heat may be transferred via gas in the cavity 5 to the die pad 21 which acts as a heat sink. The hole 212 in the die pad may allow balancing of pressure which may be beneficial in view of the heating of the heater.

## Claims

1. Method for manufacturing a gas sensor package, comprising the steps of
- mounting a semiconductor chip (3) on a carrier (2),
- applying a molding compound (1) for at least partially enclosing the semiconductor chip (3) thereby generating an opening (11) in the molding compound (1), the opening (11) providing access to a portion of the semiconductor chip (3) being uncovered by the molding compound (1),
- applying a sensitive material which is sensitive to one or more analytes, through the opening (11) in the molding compound (1) onto the uncovered portion of the semiconductor chip (3) for building a layer (31) sensitive to a gas.

2. Method according to claim 1,
wherein the semiconductor chip (3) is mounted prior to applying the molding compound (1), and
wherein the sensitive material is applied after the molding compound (1) is applied.

3. Method according to claim 1 or claim 2,
wherein multiple gas sensor packages are manufactured comprising the steps of
- mounting multiple semiconductor chips (3) onto the carrier (2) which carrier (2) is a common carrier (2) configured to accept multiple semiconductor chips (3),
- for each semiconductor chip (3) applying the molding compound (1) for at least partially enclosing the semiconductor chip (3) thereby generating an opening (11), the opening (11) providing access to a portion of the semiconductor chip (3) being uncovered by the molding compound (1), and
- for each semiconductor chip (3) applying the sensitive material through the opening (11) onto the uncovered portion of the semiconductor chip (3) for building a layer (31) sensitive to a gas.

4. Method according to any one of the preceding claims,
wherein prior to mounting the or each semiconductor chip (3) onto the carrier (2), the or each semiconductor chip (3) is prepared by
- integrating a processing circuit (36) to a front side (fs) of the semiconductor chip (3),
- integrating a heater (34) to the front side (fs) of the semiconductor chip (3),
- generating a recess (32) in a back side (bs) of the semiconductor chip (3) underneath the heater (34) thereby generating a membrane (39) in the semiconductor chip (3).

5. Method according to claim 3 in combination with claim 4,
comprising
- heating the heaters (34) of the semiconductor chips (3) while being arranged on the common carrier (2),
- in particular heating the heaters (34) by applying a current to the common carrier (2) or at least parts thereof,
- in particular wherein the current is dimensioned in time and magnitude for annealing the sensitive layers (31).

6. Method according to claim 3 or claim 5,
wherein the molding compound (1) is applied to all semiconductor chips (3) on the common carrier (2) in a common manufacturing step thereby generating a continuous molding compound (1) across the common carrier (2) .

7. Method according to any one of the preceding claims 3, 5, 6,
wherein the common carrier (2) comprises an electrically conducting leadframe (4), the leadframe (4) comprising die pads (21) for mounting the semiconductor chips (3) to, contact pads (22-27) for electrically contacting the gas sensor packages, and support leads (41, 411, 412, 42, 421) connected to the die pads (21) and the contact pads (22-27),
the method comprising the step of
applying the molding compound (1) such that the openings (11) are arranged in a front side (FS) of the molding compound (1) and that the die pads (21) and at least portions of the contact pads (22-27) are exposed from the molding compound (1) in a back side (BS) of the molding compound (1) opposite the front side (FS).

8. Method according to claim 7,
wherein the support leads (41, 411, 42, 421) connecting the contact pads (22-27) or the contact pads (22-27) are severed from the back side (BS).
and in particular wherein the support leads (411) or the contact pads (22-27) are severed by sawing.

9. Method according to claim 8,
wherein each die pad (21) has a rectangular shape each with four edges,
wherein the contact pads (22-27) are arranged at opposite edges of the corresponding die pad (21),
wherein the support leads (421) connecting each die pad (21) depart from the die pad (21) at least from one of the other two edges,
wherein the contact pads (22-27) arranged at one edge of the corresponding die pad (21) or the respective support leads (411) are severed simultaneously by sawing, and
wherein the support leads (421) connecting the die pads (21) are not severed yet.

10. Method according to claims 5 in combination with claim 9,
wherein one of the contact pads (26) serves as a pin for supplying electrical current to the heater (34) for each gas sensor chip (3) while another one (24) of the contact pads serves as a supply pin for supplying power for operating the gas sensor chip (3) except for the heater (34),
wherein for each gas sensor chip (3) arranged on the common carrier (2) the heating current is supplied to the contact pad (26) serving as heater pin.

11. Method according to claim 10,
wherein a third one (23) of the contact pads serves as a ground pin and is electrically connected to the die pad (21), and
wherein for each gas sensor chip (3) arranged on the common carrier (2) the heating current is applied between the contact pad (26) serving as heater pin and the third contact pad (23) serving as ground pin.

12. Method according to claim 5,
wherein the molding compound (1) and the common carrier (2) are diced for separating the gas sensor packages after having annealed the sensitive layers (31).

13. Method according to claim 12,
wherein the gas sensor chips (3) are tested and / or calibrated after having annealed the sensitive layers (31) and prior to dicing into separate gas sensor packages.

14. Method according to claim 8,
wherein the severing step is applied after the molding compound (1) is applied and after the sensitive material is applied.

15. Method according to any one of the preceding claims,
wherein the sensitive material is applied to the uncovered portion of the or each semiconductor chip (3) by contactless dispensing, and in particular by ink jet printing.

## Patentansprüche

1. Verfahren zur Herstellung eines Gassensorpakets, umfassend die folgenden Schritte
- Montieren eines Halbleiterchips (3) auf einen Träger (2),
- Aufbringen einer Formmasse (1) zum zumindest teilweisen Einschliessen des Halbleiterchips (3), wodurch eine Öffnung (11) in der Formmasse (1) erzeugt wird, wobei die Öffnung (11) Zugang zu einem Abschnitt des Halbleiterchips (3) bietet, der durch die Formmasse (1) freigelegt ist,
- Aufbringen eines empfindlichen Materials, das in Bezug auf einen oder mehreren Analyten empfindlich ist, durch die Öffnung (11) in der Formmasse (1) auf den unbedeckten Teil des Halbleiterchips (3) zum Aufbau einer gasempfindlichen Schicht (31).

2. Verfahren nach Anspruch 1,
wobei der Halbleiterchip (3) vor dem Aufbringen der Formmasse (1) montiert wird, und
wobei das empfindliche Material nach dem Auftragen der Formmasse (1) aufgebracht wird.

3. Verfahren nach Anspruch 1 oder Anspruch 2,
wobei mehrere Gassensorpakete hergestellt werden, umfassend die folgenden Schritte
- Montage mehrerer Halbleiterchips (3) auf dem Träger (2), wobei der Träger (2) ein gemeinsamer Träger (2) ist, der so konfiguriert ist, dass er mehrere Halbleiterchips (3) aufnimmt,
- für jeden Halbleiterchip (3) Aufbringen der Formmasse (1) zum zumindest teilweisen Einschliessen des Halbleiterchips (3), wodurch eine Öffnung (11) erzeugt wird, wobei die Öffnung (11) Zugang zu einem Abschnitt des Halbleiterchips (3) bietet, der von der Formmasse (1) freigelegt ist, und
- für jeden Halbleiterchip (3) Aufbringen des empfindlichen Materials durch die Öffnung (11) auf den unbedeckten Teil des Halbleiterchips (3) zum Aufbau einer gasempfindlichen Schicht (31).

4. Verfahren nach einem der vorangehenden Ansprüche,
wobei vor der Montage des oder jedes Halbleiterchips (3) auf den Träger (2) der oder jeder Halbleiterchip (3) vorbereitet wird durch
- Integration einer Verarbeitungsschaltung (36) auf einer Vorderseite (fs) des Halbleiterchips (3),
- Integration einer Heizung (34) an der Vorderseite (fs) des Halbleiterchips (3),
- Erzeugen einer Aussparung (32) in einer Rückseite (bs) des Halbleiterchips (3) unterhalb der Heizung (34), wodurch eine Membran (39) in dem Halbleiterchip (3) erzeugt wird.

5. Verfahren nach Anspruch 3 in Kombination mit Anspruch 4, umfassend
- Erhitzen der Heizungen (34) der Halbleiterchips (3), während diese auf dem gemeinsamen Träger (2) angeordnet sind,
- insbesondere Erhitzen der Heizelemente (34) durch Anlegen eines Stroms an den gemeinsamen Träger (2) oder zumindest Teile davon,
- insbesondere wobei der Strom zum Ausglühen der empfindlichen Schichten (31) bezogen auf Zeit und Grösse dimensioniert wird.

6. Verfahren nach Anspruch 3 oder Anspruch 5,
wobei die Formmasse (1) auf alle Halbleiterchips (3) auf dem gemeinsamen Träger (2) in einem gemeinsamen Herstellungsschritt aufgebracht wird, wodurch eine kontinuierliche Formmasse (1) über den gemeinsamen Träger (2) erzeugt wird.

7. Verfahren nach einem der vorangehenden Ansprüche 3, 5, 6,
wobei der gemeinsame Träger (2) einen elektrisch leitenden Leadframe (4) umfasst, wobei der Leadframe (4) Die-Pads (21) zum Montieren der Halbleiterchips (3), Kontaktpads (22-27) zum elektrischen Kontaktieren der Gassensorpakete und Stützleitungen (41, 411, 412, 42, 421), die mit den Die-Pads (21) und den Kontaktpads (22-27) verbunden sind, umfasst,
wobei das Verfahren den folgenden Schritt umfasst
Aufbringen der Formmasse (1) derart, dass die Öffnungen (11) in einer Vorderseite (FS) der Formmasse (1) angeordnet sind und dass die Die-Pads (21) und zumindest Teile der Kontaktpads (22-27) in einer der Vorderseite (FS) gegenüberliegenden Rückseite (BS) der Formmasse (1) aus der Formmasse (1) herausstehen.

8. Verfahren nach Anspruch 7,
wobei die Stützleitungen (41, 411, 42, 421), die die Kontaktpads (22-27) verbinden, oder die Kontaktkissen (22-27) von der Rückseite (BS) abgetrennt sind
und wobei insbesondere die Stützleitungen (411) oder die Kontaktpads (22-27) durch Sägen abgetrennt werden.

9. Verfahren nach Anspruch 8,
wobei jedes Die-Pad (21) eine rechteckige Form mit jeweils vier Kanten hat,
wobei die Kontaktpads (22-27) an gegenüberliegenden Kanten des entsprechenden Die-Pad (21) angeordnet sind,
wobei die Stützleitungen (421), die jeden Die-Pad (21) verbinden, vom Die-Pad (21) mindestens von einer der beiden anderen Kanten ausgehen,
wobei die Kontaktpads (22-27), die an einer Kante des entsprechenden Die-Pad (21) oder der jeweiligen Stützleitungen (411) angeordnet sind, gleichzeitig durch Sägen abgetrennt werden, und
wobei die Stützleitungen (421), die die Die-Pads (21) verbinden, noch nicht abgetrennt sind.

10. Verfahren nach Anspruch 5 in Verbindung mit Anspruch 9,
wobei eine der Kontaktpads (26) als ein Stift zum Zuführen von elektrischem Strom zu der Heizung (34) für jeden Gassensorchip (3) dient, während eine andere (24) der Kontaktpads als ein Zuführungsstift zum Zuführen von Energie zum Betreiben des Gassensorchips (3) mit Ausnahme der Heizung (34) dient,
wobei für jeden Gassensorchip (3), der auf dem gemeinsamen Träger (2) angeordnet ist, der Heizstrom dem als Heizstift dienenden Kontaktpad (26) zugeführt wird.

11. Verfahren nach Anspruch 10,
wobei ein dritter (23) der Kontaktpads als Erdungsstift dient und elektrisch mit dem Die-Pad (21) verbunden ist, und
wobei für jeden Gassensorchip (3), der auf dem gemeinsamen Träger (2) angeordnet ist, der Heizstrom zwischen dem als Heizstift dienenden Kontaktpad (26) und dem als Massestift dienenden dritten Kontaktpad (23) angelegt wird.

12. Verfahren nach Anspruch 5,
wobei die Formmasse (1) und der gemeinsame Träger (2) zum Trennen der Gassensorpakete nach dem Ausglühen der empfindlichen Schichten (31) in Würfel geschnitten werden.

13. Verfahren nach Anspruch 12,
wobei die Gassensorchips (3) nach dem Ausglühen der empfindlichen Schichten (31) und vor dem Zerteilen in getrennte Gassensorpakete getestet und/oder kalibriert werden.

14. Verfahren nach Anspruch 8,
wobei der Trennschritt nach dem Aufbringen der Formmasse (1) und nach dem Aufbringen des empfindlichen Materials durchgeführt wird.

15. Verfahren nach einem der vorangehenden Ansprüche,
wobei das empfindliche Material auf den unbedeckten Teil des oder jedes Halbleiterchips (3) durch kontaktloses Verteilen und insbesondere durch Tintenstrahldrucken aufgebracht wird.

## Revendications

1. Procédé de fabrication d'un ensemble de capteurs de gaz, comprenant les étapes suivantes
- monter une puce semi-conductrice (3) sur un support (2),
- appliquer un composé de moulage (1) pour enfermer au moins partiellement la puce semi-conductrice (3), générant ainsi une ouverture (11) dans le composé de moulage (1), l'ouverture (11) donnant accès à une partie de la puce semi-conductrice (3) qui est découverte par le composé de moulage (1),
- appliquer un matériau sensible, qui est sensible par rapport à un ou plusieurs analytes, par l'ouverture (11) sur la partie découverte de la puce semi-conductrice (3) pour former une couche (31) sensible à un gaz.

2. Procédé selon la revendication 1,
dans lequel la puce semi-conductrice (3) est montée avant l'application du composé de moulage (1), et
dans lequel le matériau sensible est appliqué après l'application de la masse de moulage (1).

3. Procédé selon la revendication 1 ou la revendication 2, dans lequel des ensembles de capteurs de gaz multiples sont fabriqués, comprenant les étapes suivantes
- monter plusieurs puces semi-conductrices (3) sur le support (2), lequel support (2) est un support commun (2) configuré pour accepter plusieurs puces semi-conductrices (3),
- pour chaque puce semi-conductrice (3), appliquer le composé de moulage (1) pour enfermer au moins partiellement la puce semi-conductrice (3), générant ainsi une ouverture (11), l'ouverture (11) donnant accès à une partie de la puce semi-conductrice (3) qui est découverte par le composé de moulage (1), et
- pour chaque puce semi-conductrice (3), appliquer le matériau sensible à travers l'ouverture (11) sur la partie découverte de la puce semi-conductrice (3) afin de former une couche (31) sensible à un gaz.

4. Procédé selon l'une des revendications précédentes,
dans lequel, avant de monter la ou chaque puce semi-conductrice (3) sur le support (2), la ou chaque puce semi-conductrice (3) est préparée
- en intégrant un circuit de traitement (36) à une face avant (fs) de la puce semi-conductrice (3),
- en intégrant un chauffage (34) à la face avant (fs) de la puce semi-conductrice (3),
- en créant un évidement (32) dans une face arrière (bs) de la puce semi-conductrice (3) sous le chauffage (34), générant ainsi une membrane (39) dans la puce semi-conductrice (3).

5. Procédé selon la revendication 3 en combinaison avec la revendication 4, comprenant
- chauffer les chauffages (34) des puces semi-conductrices (3) tout en étant disposées sur le support commun (2),
- en particulier chauffer les chauffages (34) en appliquant un courant au support commun (2) ou au moins à des parties de celui-ci,
- en particulier dans lequel le courant est dimensionné en temps et en amplitude pour le recuit des couches sensibles (31).

6. Procédé selon la revendication 3 ou la revendication 5,
dans lequel le composé de moulage (1) est appliqué à toutes les puces semi-conductrices (3) sur le support commun (2) dans une étape de fabrication commune, générant ainsi un composé de moulage continu (1) à travers le support commun (2).

7. Procédé selon l'une des revendications précédentes 3, 5, 6,
dans lequel le support commun (2) comprend une grille de connexion électriquement conductrice (4), la grille de connexion (4) comprenant des plots de matrice (21) pour monter les puces semi-conductrices (3), des plots de contact (22-27) pour contacter électriquement les ensembles de capteurs de gaz, et des fils de support (41, 411, 412, 42, 421) connectés aux plots de matrice (21) et aux plots de contact (22-27),
le procédé comprenant l'étape consistant à l'application de la masse de moulage (1) de telle sorte que les ouvertures (11) soient disposées dans une face avant (FS) de la masse de moulage (1) et que les plots de matrice (21) et au moins des parties des plots de contact (22-27) soient exposés de la masse de moulage (1) dans une face arrière (BS) de la masse de moulage (1) opposée à la face avant (FS).

8. Procédé selon la revendication 7,
dans lequel les fils de support (41, 411, 42, 421) reliant les plots de contact (22-27) ou les plots de contact (22-27) sont sectionnés de la face arrière (BS)
et en particulier dans lequel les fils de support (411) ou les plots de contact (22-27) sont sectionnés par sciage.

9. Procédé selon la revendication 8,
dans lequel chaque plot de matrice (21) a une forme rectangulaire avec quatre bords,
dans lequel les plots de contact (22-27) sont disposés sur des bords opposés du plot de matrice (21) correspondant,
dans lequel les fils de support (421) reliant chaque plot de matrice (21) partent du plot de matrice (21) au moins d'un des deux autres bords,
dans lequel les plots de contact (22-27) disposés sur un bord du plot de matrice (21) correspondant ou les fils de support (411) respectifs sont sectionnés simultanément par sciage, et
dans lequel les fils de support (421) reliant les plots de matrice (21) ne sont pas encore sectionnés.

10. Procédé selon les revendications 5 en combinaison avec la revendication 9,
dans lequel l'une des plots de contact (26) sert de broche pour alimenter en courant électrique le chauffage (34) pour chaque puce de capteur de gaz (3) tandis qu'une autre (24) des plots de contact sert de broche d'alimentation pour fournir l'énergie nécessaire au fonctionnement de la puce de capteur de gaz (3) à l'exception du chauffage (34),
dans lequel, pour chaque puce de capteur de gaz (3) placée sur le support commun (2), le courant de chauffage est fourni au plot de contact (26) servant de broche de chauffage.

11. Procédé selon la revendication 10,
dans lequel un troisième (23) des plots de contact sert de broche de terre et est électriquement connecté au plot de matrice (21), et
dans lequel, pour chaque puce de capteur de gaz (3) disposée sur le support commun (2), le courant de chauffage est appliqué entre le plot de contact (26) servant de broche de chauffage et le troisième plot de contact (23) servant de broche de masse.

12. Procédé selon la revendication 5,
dans lequel le composé de moulage (1) et le support commun (2) sont coupés en dés pour séparer les paquets de capteurs de gaz après avoir recuit les couches sensibles (31).

13. Procédé selon la revendication 12,
dans lequel les puces du capteur de gaz (3) sont testées et/ou calibrées après avoir recuit les couches sensibles (31) et avant de les découper en paquets séparés de capteurs de gaz.

14. Procédé selon la revendication 8,
dans lequel l'étape de séparation est appliquée après l'application du composé de moulage (1) et après l'application du matériau sensible.

15. Procédé selon l'une des revendications précédentes,
dans lequel le matériau sensible est appliqué sur la partie découverte de la ou de chaque puce semi-conductrice (3) par distribution sans contact, et en particulier par impression à jet d'encre.
